# EUROPEAN PATENT APPLICATION

(11) **EP 3 628 971 A1**
(43) Date of publication of application: **01.04.2020**
(21) Application number: 18196996.5
(22) Date of filing: 26.09.2018
(51) Int. Cl.: G01C 21/34, G01C 21/36, G01N 33/00, G06Q 10/00

(54) **A COMPUTER-IMPLEMENTED METHOD AND A SYSTEM FOR DETERMINING A ROUTE**

(71) Applicant: Valeo Systemes Thermiques-THS, 78322 Le Mesnil Saint Denis Cedex (FR)
(72) Inventor: DE PELSEMAEKER, Georges, 78322 LE MESNIL SAINT DENIS CEDEX (FR); CLEMARON, Laetitia, 78322 LE MESNIL SAINT-DENIS CEDEX (FR); DEVEYCX, Marion, 78322 LE MESNIL SAINT-DENIS (FR)
(74) Representative: Metz, Gaëlle

(57) **Abstract**

A computer-implemented method for determining a route, in particular for a vehicle, comprising: obtaining navigation data, a starting point S and a destination point D and determining 110 a plurality of routes between the starting point S and the destination D; obtaining 120 weather forecast data for the plurality of routes; obtaining 130 air pollution forecast data for the plurality of routes; obtaining 140 user preference information; determining 150 route score, based on user preference information, for each of the plurality of routes without taking into account the weather forecast data and the air pollution forecast data, so that a first group of routes is obtained; determining 160 route score, based on user preference information, for each of the plurality of routes with taking into account the weather forecast data and the air pollution forecast data, so that a second group of routes is obtained; outputting 170 a first route candidate R1 and a second route candidate R2, wherein the first route candidate R1 is the candidate with optimal route score from the first group of routes and the second route candidate R2 is the candidate with optimal route score from the second group of routes.

## Description

### FIELD OF THE INVENTION

The invention relates to determination of a route. In particular, it relates to determination of a plurality of routes in an environment subject to air pollution.

### BACKGROUND OF THE INVENTION

Air pollution is a serious concern for people because of its harmful effects to health - both in physical and psychological sense. Passengers travelling in vehicles are especially at risk, as the vehicles are part of traffic existing in urbanized areas, which tend to have higher concentration of air pollution and higher levels of pollutant gases and particles. Naturally, people commuting on foot, on bicycles or motorcycles are even more concerned.

Pollution levels are monitored by government agencies and private entities. The measurements are often provided in form of so called pollution maps. For example, a layer of visually represented information is added to a classic map, so that pollution concentration within a specific area or region can be determined and evaluated. Such maps usually have a set time-resolution, which means that the information is updated for example once an hour or once a day. Pollution map data is obtained for example by measuring pollution with a number of measuring stations or pollution measurement devices of a fixed position.

A typical navigation system, for example vehicle navigation system, is configured to propose one or more route candidates between a starting point and selected destination point. The routes are usually ranked based on several criteria, inter alia distance, time or cost.

### SUMMARY OF THE INVENTION

The object of the invention is, among others, a computer-implemented method for determining a route, in particular for a vehicle, comprising: obtaining navigation data, a starting point and a destination point and determining a plurality of routes between the starting point and the destination; obtaining weather forecast data for the plurality of routes; obtaining air pollution forecast data for the plurality of routes; obtaining user preference information; determining route score, based on user preference information, for each of the plurality of routes without taking into account the weather forecast data and the air pollution forecast data, so that a first group of routes is obtained; determining route score, based on user preference information, for each of the plurality of routes with taking into account the weather forecast data and the air pollution forecast data, so that a second group of routes is obtained; outputting a first route candidate and a second route candidate, wherein the first route candidate is the candidate with optimal route score from the first group of routes and the second route candidate is the candidate with optimal route score from the second group of routes.

Preferably, the air pollution forecast data is at least partly obtained from a pollution map from a data supplier.

Preferably, the air pollution forecast data is at least partly obtained from the vehicle's onboard pollution sensors.

Preferably, the air pollution forecast data and/or weather forecast data is at least partly obtained from the vehicle's system information source comprising at least one from the group of: wipers speed, lighting operation, braking efficiency.

Preferably, the air pollution forecast data and/or weather forecast data is at least partly obtained from at least one from the group of: meteorological stations, pollution stations with a fixed location, a personal mobile pollution sensors (pollution pods), other vehicles, a satellite, a weather map.

Preferably, the method is iterated based on elapsed time or based on a travelled distance.

Preferably, the method is iterated for each update of air pollution forecast data.

Preferably, the route score is determined based on at least one criteria selected from the group: distance, duration, cost.

Preferably, the route score for each route of the second group of routes is calculated based on at least one criteria selected from the group: sun exposure, pollution level, allergen exposure, driving conditions.

Another object of the invention is a system comprising: communication means configured to obtain navigation data, starting point and destination point and plurality of routes between the starting point and destination point, weather forecast data for the plurality of routes, air pollution forecast data for the plurality of routes, user preference information; control means configured to determine route score, based on user preference information, for each of the plurality of routes without taking into account the weather forecast data and the air pollution forecast data so that a first group of routes is obtained; and to determine route score, based on user preference information, for each of the plurality of routes with taking into account the weather forecast data and the air pollution forecast data so that a second group of routes is obtained; output means configured to output a first route candidate and a second route candidate, wherein the first route candidate is the candidate with optimal route score from the first group of routes and the second candidate is the candidate with optimal route score from the second group of routes.

Preferably, the system further comprises pollution sensing means adapted to be mounted on a vehicle, configured to provide real-time pollution measurement data, indicative of pollution levels within the vehicle cabin and/or outside the vehicle's cabin, in its immediate vicinity.

Preferably, the communication means are configured to obtain the air pollution forecast data and/or weather forecast data at least partly obtained from the vehicle's system information source comprising at least one from the group of: wipers speed, lighting operation, braking efficiency.

Another object of the invention is a computer readable storage medium storing a program of instructions executable by a machine to perform a method as described above.

### BRIEF DESCRITPTION OF DRAWINGS

Examples of the invention will be apparent from and described in detail with reference to the accompanying drawings, in which:
Fig. 1 shows a flowchart presenting the method according to the invention;
Fig. 2 shows an example of outputted route candidates;
Fig. 3 shows a schematic representation of a system according to invention.

### DETAILED DESCRIPTION OF EMBODIMENTS

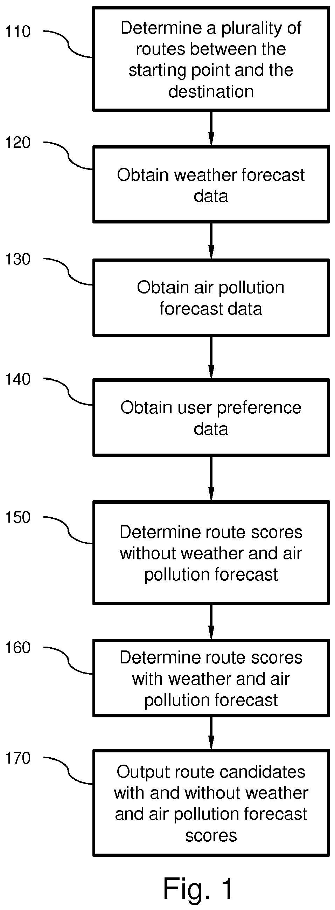

Fig. 1 shows a flowchart presenting the method according to the invention. Firstly, the method comprises determining 110 a plurality of routes between a starting point S and a destination D. For this purpose navigation data, starting point S and destination point D are obtained. The starting point S can be input manually by the user through user interface or can be obtained from the navigation data automatically, by known means.

Next, the method comprises obtaining 120 weather forecast data for the plurality of routes and obtaining 130 air pollution forecast data for the plurality of routes.

The weather forecast data concerns most recent weather conditions and forecasted weather conditions. It can include temperature, barometric pressure, wind speed, sun exposure, humidity, visibility, time of sunrise and sunset, UV index etc. Information on current and forecasted weather conditions may be taken into account in view of its influence on safety and/or attractiveness to the user. Some weather conditions may imply worse driving conditions, which in turn imply worse safety.

The air pollution forecast data concerns most recent pollution levels and forecasted pollution levels. Preferably, it is at least partly obtained from a pollution map from a data supplier. Such pollution map data can comprise information on particulate matter levels, carbon monoxide, sulfur oxides, nitrogen oxides, ozone, spread over a defined geographical area. It is also envisaged that this pollution map data may be supplemented with information on pollens and allergens.

Additionally, the air pollution forecast data may be at least partly obtained from the vehicle's onboard pollution sensors. In particular, these sensors can output data indicative of pollution levels outside the vehicle cabin, in the immediate vicinity of the vehicle. The data can be then used for real-time adjustment of the forecast, e.g. using machine learning models.

In one option, the air pollution forecast data and/or weather forecast data is at least partly obtained from the vehicle's system information source comprising at least one from the group of: wipers speed, lighting operation, braking efficiency. In other words, the data is obtained in an indirect manner from the vehicle on-board information system. For example, a currently set wipers speed may be indicative of raining intensity. The set lightening parameters may be indicative of visibility in front of the vehicle. The measured braking efficiency may be indicative of road conditions, and for example be correlated with rain, ground frost etc.

In one option, the air pollution forecast data and/or weather forecast data is at least partly obtained from at least one from the group of: meteorological stations, pollution stations with a fixed location, a personal mobile pollution sensors (pollution pods), other vehicles, a satellite, an open source pollution or weather maps.

In the next step, the method comprises obtaining 140 user preference information. The user preference information may relate for example to the distance, duration or a cost of the trip. These preferences may be grouped into a first group of preferences. The user preference information may relate for example also to pollution levels and weather. These preferences may be grouped into a second group of preferences. In particular, the second group of preferences may relate to sun exposure, pollution level, allergen exposure, safety (driving conditions). For example, the user may input preference information indicating preference of a route with maximized sun exposure, with limited cloud coverage of the sky, with minimized pollution level. Alternatively, the user may input preference information indicating preference of a route with good driving conditions and limited sun exposure.

It is also envisaged that a third group of preferences may be formed, which could relate to attractiveness of the route in view of preferred sport activities, points of interest exposure, nature/urban ratio exposure, type of terrain etc.

In the next step, the method comprises determining 150 route score, based on user preference information, for each of the plurality of routes without taking into account the weather forecast data and the air pollution forecast data, so that a first group of routes is obtained. In other words, a first route score is determined for each of the plurality of routes only based on the first group of preferences. What follows is that, preferably, the route score is determined based on at least one criteria selected from the group: distance, duration, cost - in a known manner.

In the next step, the method comprises determining 160 route score, based on user preference information, for each of the plurality of routes with taking into account the weather forecast data and the air pollution forecast data, so that a second group of routes is obtained. In other words, a second route score is determined for each of the plurality of routes based on the first group of preferences and the second group preferences. What follows is that, preferably, the route score is additionally determined based on at least one criteria selected from the group: sun exposure, pollution level, allergen exposure, safety (driving conditions).

Analogously, if a third group of preferences is present, the method can comprise determining route score for each of the plurality of routes based on the first group of preferences, the second group preferences and the third group of preferences, so that a third group of routes is obtained.

In the next step, the method comprises outputting 170 a first route candidate R1 and a second route candidate R2, wherein the first route candidate R1 is the candidate with optimal route score from the first group of routes and the second route candidate R2 is the candidate with optimal route score from the second group of routes. The determination of the optimal candidate in both cases can be carried out by known optimal route determination algorithms. The optimal route score in both cases may be for example that which corresponds most closely to the selected preference information parameters. The second route candidate R2 in any case needs to take into account more parameters than the first route candidate R1.

Analogously, if a third group of preferences is present, the method can comprise outputting a third route candidate which is the candidate with optimal route score from third group of routes.

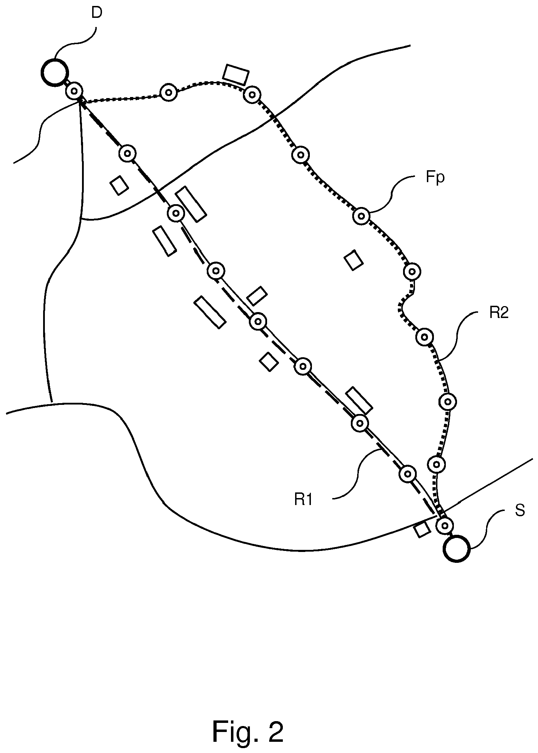

Preferably, part of the steps or all the steps can be iterated based on elapsed time or based on a travelled distance. Alternatively or additionally, the method can be iterated for each update of air pollution forecast data. The iterations are explained in relation to Fig. 2

The above-described method can be carried out by means of cloud computing. In particular, at least part of the steps can be carried out in the cloud, with transmitting the route candidates to the user.

Fig. 2 shows an example of outputted route candidates. A starting point S and a destination point D are obtained. In this case, the starting point S is the current geographical position of the vehicle. A plurality of routes is available between the starting point S and the destination point D. It is shown in Fig. 2 that a first route candidate R1 and a second route candidate R2 are outputted. The first route candidate R1 is shorter and could take less time to travel between points S and D. However, based on additional, second group of preferences, the second route candidate R2 is outputted together with the first route candidate R1. The user can thus choose between the route candidates, while having more insight about the consequences of the choice.

In Fig. 2 the frequency of iterations of the method or its selected steps along the routes R1, R2 can be observed - the occurrences of iterations are visualised by means of update points Fp. When the vehicle reaches these points along the routes R1, R2, an iteration of the method or its selected steps takes place and the updated data is obtained. In particular, the updated pollution and/or weather forecast data can be obtained when the vehicle is at update points Fp to propose any new optimal route candidates, which take into account new information. The iteration, as explained above, can be based preferably on air pollution forecast data update rate. However, the distribution of update points Fp can be also based on elapsed time, e.g. once every minute for trips calculated for less than 1 hour and once every ten minutes for longer trips. The distribution of update points Fp can be also based on a travelled distance or traffic information update from the navigation service provider or other basis. For obtaining optimal second route candidate R2, the AQI value, a weather index or others can be calculated for each point, then combined together to form scores for each point and then the average score is calculated for each route. Next, the route with the best (e.g. highest) average is selected as the optimal second route candidate R2. In any case, the second route candidate R2 is a compromise between the best score and additional time with respect to the first route candidate R1. It is envisaged that the second route candidate is constrained to add for example 10% of travelling time for short trips which do not exceed 30 minutes, 5% for trips between 30min and 2h and then 2% for longer trips. In other words, the second route candidate R2 is constrained to a specific range of additional time with respect to the first route candidate R1.

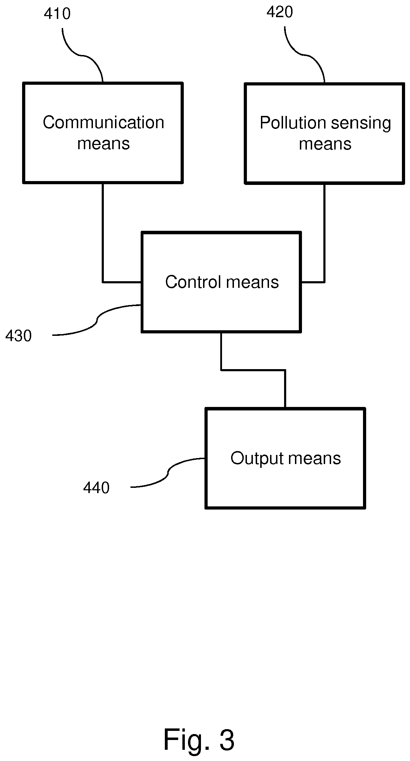

Fig. 3 shows a schematic representation of a system according to invention. The system comprises communication means 410, control means 430 and output means 440. The system comprises communication means 410 configured to obtain navigation data, a starting point S and destination point D and plurality of routes between the starting point S and destination point D. This may be achieved in a conventional manner known in the art, by means of global positioning system integration, optionally coupled with internet connection to navigation services provider. Preferably, the communication means 410 are adapted to provide internet connection for the system, in particular to cloud computing services.

The communication means 410 are further adapted to obtain weather forecast data and air pollution forecast data for the plurality of routes. In particular, the communication means 410 may be adapted to obtain air pollution forecast data for example in form of the pollution map data, which is indicative of most current air pollution levels within the specified area and a forecast for air pollution levels in the future, from a data supplier.

Further, the communication means 410 are adapted to obtain user preference information from the user. This may be done by integration with any known input means, which might be a part of output means 440, e.g. by means of a touch screen of the vehicle entertainment system or through an application on a smart phone.

The control means 430 are configured to determine route score, based on user preference information, for each of the plurality of routes without taking into account the weather forecast data and the air pollution forecast data so that a first group of routes is obtained; and to determine route score, based on user preference information, for each of the plurality of routes with taking into account the weather forecast data and the air pollution forecast data so that a second group of routes is obtained.

The output means 440 are configured to output a first route candidate R1 and a second route candidate R2, wherein the first route candidate R1 is the candidate with optimal route score from the first group of routes and the second candidate R2 is the candidate with optimal route score from the second group of routes.

Preferably, the system further comprises pollution sensing means 420 adapted to be mounted on a vehicle and to be configured to provide real-time pollution measurement data, indicative of pollution levels within the vehicle cabin and/or outside the vehicle's cabin, in its immediate vicinity. This data may be transmitted using communication means 410 to the data supplier or used internally to supplement available data.

Preferably, the communication means 410 are configured to obtain the air pollution forecast data and/or weather forecast data which is at least partly obtained from the vehicle's system information source comprising at least one from the group of: wipers speed, lighting operation, braking efficiency.

Thanks to an anticipation model using weather, industrial pollution and traffic forecast based on machine learning model, the data could be adjust for the coming kilometers in real time thanks to on-board sensor.

It is understood that embodiments of the present invention can be carried out in form of hardware, software or a combination of both. Any such software can be stored in the form of a volatile or non-volatile storage, for example a storage like a ROM, whether erasable or rewritable or not, or in the form of memory such as, for example RAM, memory chips, device or integrated circuits or an optically or magnetically readable medium such as, for example, a CD, DVD, magnetic disk or magnetic tape. It is understood that the storage devices and storage media are embodiments of machine-readable storage that are suitable for storing program or programs that, when executed, implement embodiments of the present invention. Accordingly, embodiments provide a program comprising code for implementing a system or method as described and a machine readable storage storing such a program. Further, embodiments of the present invention may be conveyed electronically via any medium such as a communication signal carried over a wired or wireless connection and embodiments suitably encompass the same.

Other variations to the disclosed embodiments can be understood and effected by those skilled in the art in practicing the claimed invention, from a study of drawings, the disclosure, and the appended claims. The mere fact that certain measures are recited in mutually different dependent claims does not indicate that a combination of these measures cannot be used to the advantage.

## Claims

1. A computer-implemented method for determining a route, in particular for a vehicle, comprising:
- obtaining navigation data, a starting point S and a destination point D and determining 110 a plurality of routes between the starting point S and the destination D;
- obtaining 120 weather forecast data for the plurality of routes;
- obtaining 130 air pollution forecast data for the plurality of routes;
- obtaining 140 user preference information;
- determining 150 route score, based on user preference information, for each of the plurality of routes without taking into account the weather forecast data and the air pollution forecast data, so that a first group of routes is obtained;
- determining 160 route score, based on user preference information, for each of the plurality of routes with taking into account the weather forecast data and the air pollution forecast data, so that a second group of routes is obtained;
- outputting 170 a first route candidate R1 and a second route candidate R2, wherein the first route candidate R1 is the candidate with optimal route score from the first group of routes and the second route candidate R2 is the candidate with optimal route score from the second group of routes.

2. A method according to claim 1, wherein the air pollution forecast data is at least partly obtained from a pollution map from a data supplier.

3. A method according to claim 1 or 2, wherein the air pollution forecast data is at least partly obtained from the vehicle's onboard pollution sensors.

4. A method according to any preceding claim, wherein the air pollution forecast data and/or weather forecast data is at least partly obtained from the vehicle's system information source comprising at least one from the group of: wipers speed, lighting operation, braking efficiency.

5. A method according to any preceding claim, wherein the air pollution forecast data and/or weather forecast data is at least partly obtained from at least one from the group of: meteorological stations, pollution stations with a fixed location, a personal mobile pollution sensors (pollution pods), other vehicles, a satellite, a weather map.

6. A method according to any preceding claim, wherein the method is iterated based on elapsed time or based on a travelled distance.

7. A method according to any preceding claim, wherein the method is iterated for each update of air pollution forecast data.

8. A method according to any preceding claim, wherein the route score is determined based on at least one criteria selected from the group: distance, duration, cost.

9. A method according to any preceding claim, wherein the route score for each route of the second group of routes is calculated based on at least one criteria selected from the group: sun exposure, pollution level, allergen exposure, driving conditions.

10. A system comprising:
- communication means 410 configured to obtain navigation data, starting point and destination point and plurality of routes between the starting point and destination point, weather forecast data for the plurality of routes, air pollution forecast data for the plurality of routes, user preference information;
- control means 430 configured to determine route score, based on user preference information, for each of the plurality of routes without taking into account the weather forecast data and the air pollution forecast data so that a first group of routes is obtained; and to determine route score, based on user preference information, for each of the plurality of routes with taking into account the weather forecast data and the air pollution forecast data so that a second group of routes is obtained;
- output means 440 configured to output a first route candidate and a second route candidate, wherein the first route candidate is the candidate with optimal route score from the first group of routes and the second candidate is the candidate with optimal route score from the second group of routes.

11. System according to claim 10, wherein it further comprises pollution sensing means 420 adapted to be mounted on a vehicle, configured to provide real-time pollution measurement data, indicative of pollution levels within the vehicle cabin and/or outside the vehicle's cabin, in its immediate vicinity.

12. System according to claim 10, wherein the communication means 410 are configured to obtain the air pollution forecast data and/or weather forecast data at least partly obtained from the vehicle's system information source comprising at least one from the group of: wipers speed, lighting operation, braking efficiency.

13. A computer readable storage medium storing a program of instructions executable by a machine to perform a method according to any of claims 1-9.
